# EUROPEAN PATENT SPECIFICATION

(11) **EP 0 470 312 B1**
(45) Date of publication and mention of the grant of the patent: **08.02.1995**
(21) Application number: 90308841.7
(22) Date of filing: 10.08.1990
(51) Int. Cl.: C12N 9/02, C12P 7/40

(54) **Novel omega-carboxyalcohol oxidase**
Omega-Carboxyalkohol Oxydase
Oméga-carboxyalcool oxydase

(43) Date of publication of application: 12.02.1992
(73) Proprietor: ASAHI KASEI KOGYO KABUSHIKI KAISHA, Osaka (JP)
(72) Inventor: Imamura, Shigeyuki, Tagata-gun, Shizuoka (JP); Muto, Naoki, Tagata-gun, Shizuoka, (JP); Ishizawa, Kenya, Mishima-shi, Shizuoka, (JP)
(74) Representative: Woods, Geoffrey Corlett

(56) References cited:
- DD-A- 239 005
- DD-A- 239 045
- US-A- 4 349 633
- BIOCHIMICA ET BIOPHYSICA ACTA, vol. 46, no. 1, 1 January1961, pages 45-50; M.A. MITZ et al.: "Omega hydroxy fatty acid dehydrogenase"
- METHODS IN ENZYMOLOGY, vol. 71, part C, 1981, pages411-420, New York, US; P.E. KOLATTUKUDY et al.: "w-Hydroxy Fatty Acid: NADP Oxidoreductase from Wound-Healing Potato Tuber Disks"
- JOURNAL AMERICAN OIL CHEM. SOC. vol. 61, no. 2, February1984, pages 447-453, US; C. RATLEDGE: "Microbial conversions of Alkanes andFatty Acids

## Description

This invention relates to an ω-carboxyalcohol oxidase, its production, a method for detecting an aliphatic alcohol or an aliphatic aldehyde or ω-carboxylic acid derivative thereof and a process for the production of a carboxylic acid.

A known oxidase having substrate specificity for an aliphatic acid is alcohol oxidase (EC. 1.1.3.13), which catalyses the following reaction to form an aldehyde:

R''' - CH₂OH + O₂ → R''' - CHO + H₂O₂

Alcohol oxidases from Basidiomycete [Biochim. Biophys. Acta, 151 : 330-342 (1968), Biochem. Biophys. Res. Commun., 20 : 630-634 (1965)], H. polymorpha, a yeast grown in methanol, and Kloeckera sp. [Agr. Biol. Chem., 36 : 2297-2306 (1972), Eur. J. Biochem., 36: 250-256 (1973), ibid., 64 : 341-350 (1976, Agr. Biol. Chem., 36 : 68-75 (1972)] are known. Alcohol oxidase from Basidiomycete has substrate (R''' -CH₂OH) specificity for a C₂₋₄ straight chain primary alcohol, allylalcohol or 2 propione-1-ol; that from H. polymorpha has substrate specificity for a C₁₋₄ straight chain primary alcohol, 2-propene-1-ol or 2-butene-1-ol; and that from Kloeckera sp. has substrate specificity for a C₁₋₄ straight chain primary alcohol, 2-propene-1-ol, 2-butene-1-ol or allylalcohol.

Hence alcohol oxidases from these sources generate aldehydes and have substrate specificity, in relation at least to straight chain primary alcohols, for lower aliphatic alcohols having less than four carbon atoms.

Enzymes having substrate specificity for glycerol, e.g. glycerol dehydrogenase or glycerol-2-dehydrogenase (EC. 1.1.1.6, EC. 1.1.1.72, EC. 1.1.1.156) and glycerol oxidase are also known. These enzymes oxidize glycerol and generate dihydroacetone or glyceroaldehyde. They do not catalyse a reaction which generates a carboxylic acid derivative.

An oxidase which has substrate specificity for a fatty acid having a hydroxy group at the ω-carboxylalcohol) is also known, which oxidizes said substrate to generate a dibasic acid. This can be obtained from animal tissue [Biochim. Biophys. Acta, 46 : 45-50 (1961)] and plant tissue [Methods in Enzymology, 71 : 411-420, (1981)]. These enzymes require essentially coenzyme NAD or NADP.

DD-A-239,005 discloses alcohol oxidases. This document indicates that the oxidases react with ethanol as a substrate.

Accordingly known alcohol oxidases have substrate specificities for lower alcohols with less than four carbon atoms, at least in relation to straight chain aliphatic primary or secondary alcohols, for glycerol, or for an ω-hydroxy carboxylic acid with coenzyme NAD or NADP. An enzyme which does not require a coenzyme, has no substrate specificity for a straight chain primary alcohol with two or less carbon atoms and which requires oxygen to generate hydrogen peroxide is not known.

We have found that Streptomyces strain AC 8205 FERM BP-2491 isolated from a soil sample from a field in Sumotoshi, Hyogo-ken, Japan, produces an ω-carboxyalcohol oxidase enzyme. We have isolated and purified said enzyme, and provided a method for the production of a carboxylic acid using said enzyme.

Said enzyme is novel in view of its enzyme action, substrate specificity and utilization of coenzyme. It has been designated ω-carboxyalcohol oxidase.

The taxonomical properties of Streptomyces strain AC 8205 are as follows:

### I. Morphological properties:

Morphological observations by optical microscopy and electron microscopy upon culturing on inorganic salt-starch agar medium, glycerol-asparagine agar medium or yeast extract-malt extract agar medium at 30°C for 14 days are as follows:
The substrate mycelium is curved or straight, grown with branching, 0.3 ∼ 0.5 »m in diameter and does not form fragmentation.

The aerial mycelium grown on substrate mycelium is formed with a curved or straight long principal axis and short branches which are alternate or opposite, 0.4 ∼ 0.6 »m in diameter. The tops of branches are loose spirals with 2 ∼ 4 rounds and approximately 10∼20 chain spores.

The spores are oval and 0.6 ∼ 0.8 x 0.8 ∼ 1.0 »m in size with spiny surfaces.

No sporangia, motile spores, verticillae and sclerotia are formed.

### II. Composition of diaminopimelic acid

LL-type diaminopimelic acid is found and no mesotype is detected according to the method of Staneck, et al [Appl. Microbiol., 28 : 226-231 (1974)].

### III. Cultural observation:

Observations on various media cultured at 30°C for 20 days are shown in Table 1.

Colour indications are made by consulting the "Color Harmony Manual", 4th Ed., 1958 (Container Corp. of America).

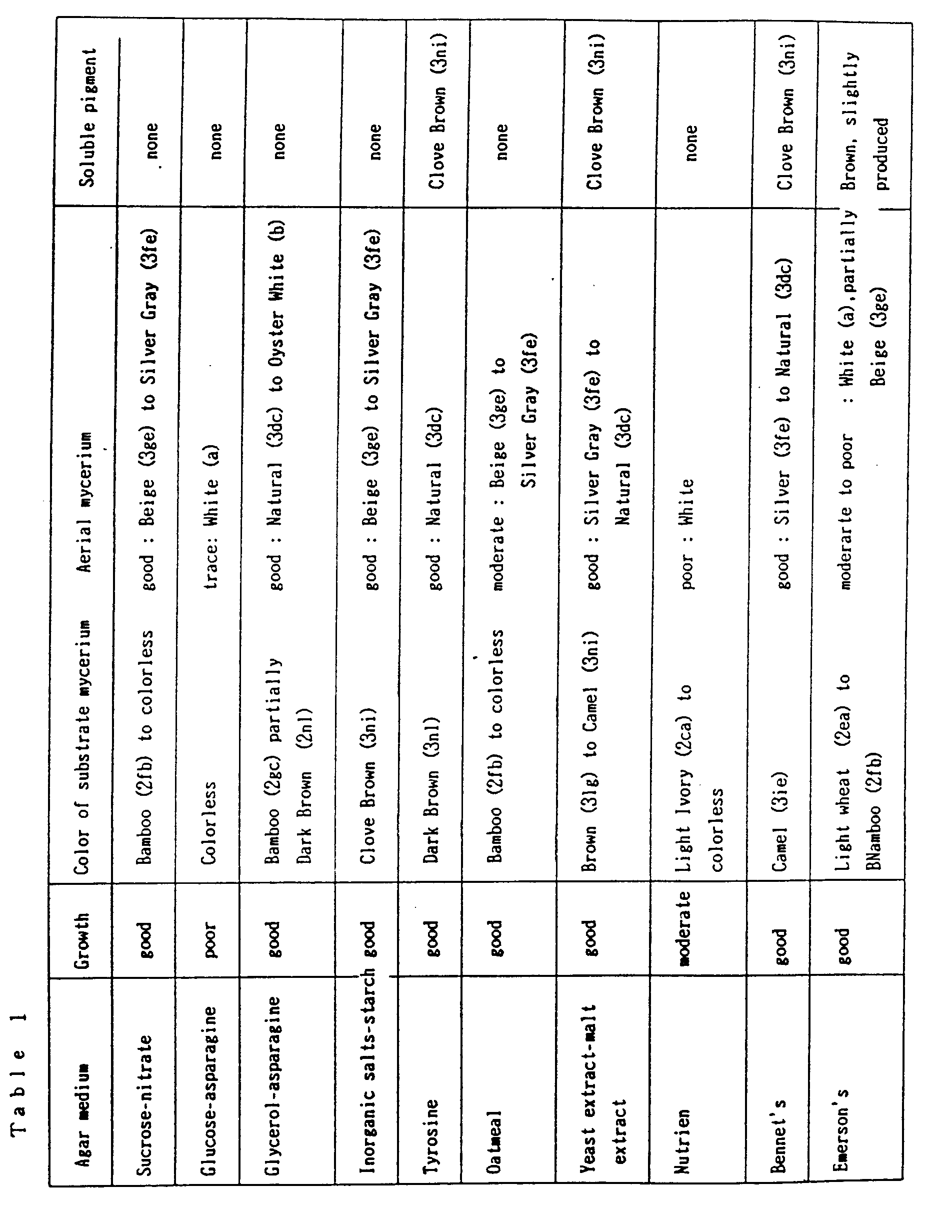

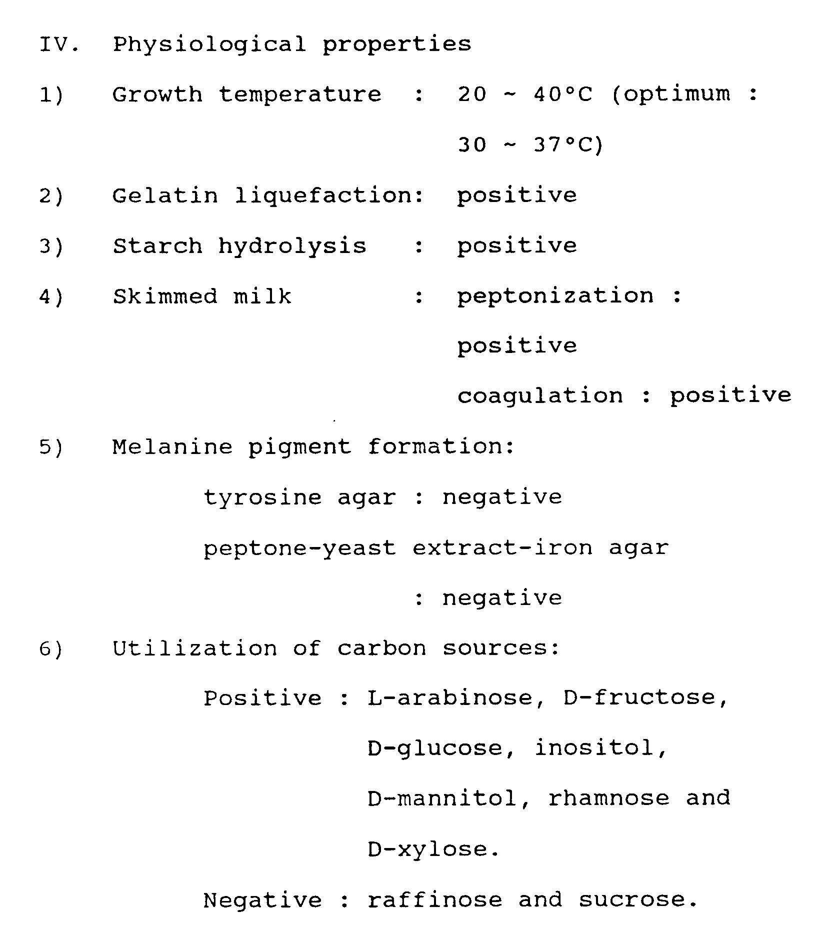

As stated above, strain AC 8205 has the taxonomical properties of forming aerial mycelium which form spores of chains with spirals on the top from the substrate mycelium and no formation of flagellate spores or sporangia. It is thus identified as belonging to genus Streptomyces. This strain is referred as Streptomyces sp. AC 8205, and was deposited on 28 June 1989 in the Fermentation Institute, Agency of Industrial Science and Technology, M.I.T.I., Japan under deposit No. FERM BP-2491.

The present invention provides an ω-carboxyalcohol oxidase having at least the following biochemical properties:
1) Enzyme action: at least catalysing the following reactions a) and b)
   a) R - CH₂OH + O₂ → R-CHO + H₂O₂
   b) R - CHO + O₂ + H₂O → R-COOH + H₂O₂
   wherein R-CH₂OH is an aliphatic alcohol except methanol and ethanol or an ω-carboxylic acid derivative of said alcohol, R-CHO is a corresponding aliphatic aldehyde or an ω-carboxylic acid derivative thereof, and R-COOH is a corresponding compound thereto;
2) substrate specificity: at least having substrate specificity for HO₂C-(CH₂)₁₁-OH, H₃C-(CH₂)₁₁-OH, H₃C-(CH₂)₉-OH, H₃C-(CH₂)₇-OH and H₃C(CH₂)₅-OH, and having no substrate specificity for methanol, ethanol and glycerol; and
3) utilization of coenzyme: no utilization of NAD and NADP.

The ω-carboxyalcohol oxidase may also have the following biochemical properties:
- molecular weight : 66000 ± 6000 (gel filtration method using a Superose 12 column),
- optimum pH : 7.5 ± 0.5
- stable pH range : 6 ∼ 9
- heat stability : stable at 45°C (at pH 7.5, for 10 minutes)
The present invention also provides a process for production of an ω-carboxyalcohol oxidase as defined above which comprises culturing a said ω-carboxyalcohol oxidase belonging to the genus Streptomyces in a medium and obtaining said ω-carboxyalcohol oxidase therefrom.

The microorganism Streptomyces sp. AC 8205 is merely illustrative of microorganisms which produce the ω-carboxyalcohol oxidase of the present invention. Other ω-carboxyalcohol oxidase-producing microorganisms belonging to the genus Streptomyces can be used. Microorganism strains are easily mutated naturally or artificially, and hence these mutants which have an ability to produce an ω-carboxyalcohol oxidase in an isolatable amount can be used in the present invention. Furthermore, strains which have been improved by recombinant DNA technology for said enzyme production are also included in the present invention.

In the accompanying drawings:
Fig. 1 is a graph showing the generation and consumption of aldehyde;
Fig. 2 is a graph the optimum pH;
Fig. 3 is a graph showing the stable pH-range;
Fig. 4 is a graph showing the heat-stability; and
Fig. 5 is a graph showing the standard curve of 12-hydroxydodecanic acid.

An embodiment of the process for the production of an ω-carboxylalcohol oxidase by the said enzyme producing microorganisms belonging to the genus Streptomyces is as follows:
An ω-carboxylalcohol oxidase-producing microorganism belonging to the genus Streptomyces is conventionally cultured in a nutrient medium for antibiotic or enzyme production. Solid or liquid culture can be used.

The nutrient sources for the microorganisms are conventional media for microorganism cultivation. As nitrogen sources, assimilable nitrogen sources, for example, corn steep liquor, soy bean powder, peptone, various meat extracts, yeast extracts, ammonium sulfate or ammonium chloride can be used. As carbon sources, assimilable carbon sources such as sucrose, glucose, molasses or glycerin can be used. Furthermore, various inorganic salts such as sodium chloride, potassium chloride, magnesium sulfate, potassium phosphate or potassium dihydrogenphosphate can optionally be used.

The culturing temperature can be varied within the range suitable for ω-carboxylalcohol oxidase production and microorganism growth. It is usually 20 to 40°C.

The culturing time can be varied according to conditions. It is usually 50 - 100 hours. The cultivation should naturally be terminated at the time of maximum production of the enzyme.

The ω-carboxylalcohol oxidase is an endo-enzyme and is included in the cells.

The ω-carboxylalcohol oxidase can be isolated by separating the cultured cells from the cultured broth, suspending the wet cells in a buffer solution such as phosphate buffer or Tris-HCl buffer, and treating by means of a French press, ultrasonication, a grinding mill treatment or a lysozyme treatment to obtain a crude solution of the ω-carboxylalcohol oxidase. The solution is further treated by known isolation and purification methods for proteins and enzymes to obtain purified ω-carboxylalcohol oxidase. For example, the enzyme solution is treated with protamine sulfate to remove nucleic acids and is subjected to organic solvent precipitation by adding an organic solvent such as acetone, methanol, ethanol or isopropanol, or salting-out by adding ammonium sulfate, and to chromatography using an ion-exchanger such as diethylaminoethyl cellulose or diethylaminoethyl Sepharose, or gel filtration chromatography using dextran gel or polyacrylamide gel. A purified enzyme powder can be obtained by combining the above procedures and finally lyophilizing the enzyme solution, with the addition of one or more stabilizer such as BSA, gelatine, amino acid, sucrose, glycerin or ethylene glycol.

Examples of an assay method and biochemical properties of an ω-carboxylalcohol oxidase of the present invention are as follows:

### 1) Assay method:

| | |
|---|---|
| 0.1 M Tris-HCl buffer (pH 7.5) | 0.20 mℓ |
| 10 mM 12-hydroxydodecanic acid | 0.05 mℓ |
| 1% Triton X-100 | 0.05 mℓ |
| 0.2% phenol | 0.05 mℓ |
| 0.3% 4-aminoantipyrine | 0.05 mℓ |
| peroxidase (50 U/mℓ) | 0.05 mℓ |
| water | 0.05 mℓ |

An enzyme solution (25 »ℓ) is added to the above mixture (0.5 mℓ), incubated at 37°C for exactly 10 minutes, and sodium dodecyl sulfate (SDS) (0.5 mℓ) is added thereto. Absorbance at 545 nm is then measured. A unit (one unit) of enzyme is defined by the activity which generates 1 »mole of hydrogen peroxide per minute.

### 2) Enzyme action:

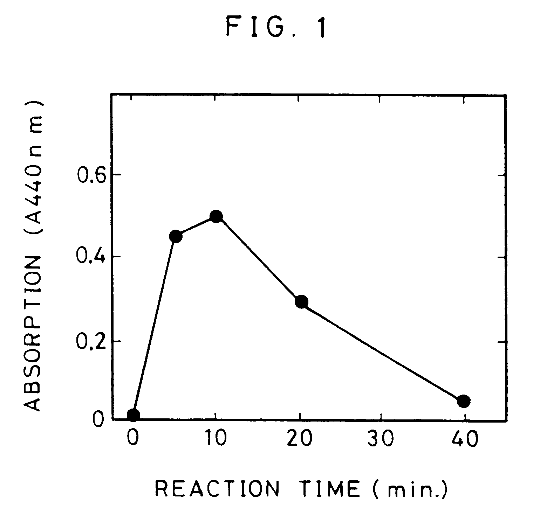

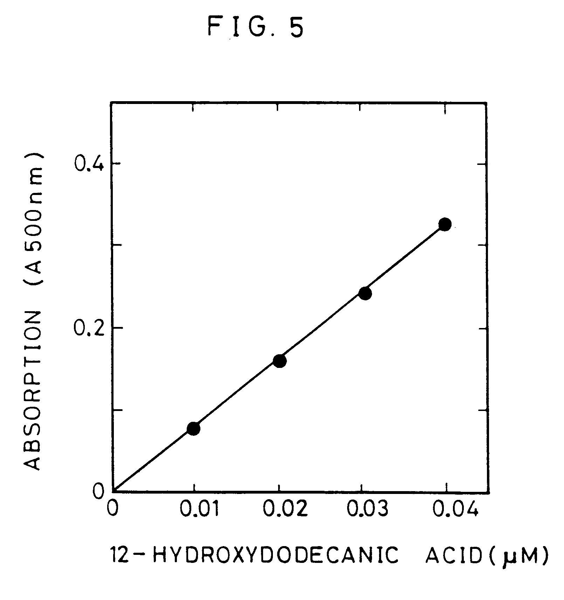

(1) Variation of the amount of aldehyde is measured by the following method:
Purified enzyme (0.3 units) is added to a reaction mixture (0.5 mℓ) consisting of 0.2 M Tris-HCl buffer (pH 8.0)(0.2 mℓ), 10 mM 12-hydroxydodecanic acid (0.01 mℓ), catalase (bovine liver, 20 units) and purified water (0.29 mℓ) and the mixture is incubated at 37°C. 12% trichloroacetic acid (0.5 mℓ) is added after 5, 10, 20 and 40 minutes, and then 0.1% 2,4-dinitrophenyl hydrazine (2N-HCl) is added therein. The mixture is subsequently centrifuged at 3000 rpm for 10 minutes. The supernatant is boiled at 100°C for 5 minutes, cooled, 1.2 N NaOH (3 mℓ) is added, mixed well, and then the absorbance at 440 nm is measured.
The result is shown in Fig. 1, in which generation of aldehyde is observed and the amount of aldehyde decreases according to the progress of the reaction.
(2) As calculated from Fig. 5, two moles of hydrogen peroxide are generated from one mole of the substrate.
This shows that the enzyme of the present invention catalyses at least the above reactions a) or b).
Examples of aliphatic alcohols except methanol and ethanol are aliphatic alcohols with more than three carbon atoms.
(3) Substrate specificity:
The substrate specificity of an enzyme is shown in Table 2. The enzyme has substrate specificity at least for HO₂C-(CH₂)₁₁-OH, H₃C-(CH₂)₁₁-OH, H₃C-(CH₂)₉-OH, H₃C-(CH₂)₇-OH and H₃C-(CH₂)₅-OH, and has no specificity to methanol, ethanol and glycerol.
Assay method: In the assay method illustrated in (1) hereinabove, 12-hydroxydodecanic acid is replaced by the substrates in Table 2.

**TABLE 2**

| Substrate | Relative activity (%) |
|---|---|
| HO₂C-(CH₂)₁₁-OH | 100 |
| HO₂C-(CH₂)₁₅-OH | 65.5 |
| H₃C-(CH₂)₇-CH=(CH₂)₈-OH | 59.1 |
| H₃C-(CH₂)₁₅-OH | 60.2 |
| H₃C-(CH₂)₁₃-OH | 76.1 |
| H₃C-(CH₂)₁₁-OH | 86.7 |
| H₃C-(CH₂)₉-OH | 87.6 |
| H₃C-(CH₂)₇-OH | 88.5 |
| H₃C-(CH₂)₅-OH | 88.5 |
| H₃C-(CH₂)₃-OH | 76.1 |
| H₃C-(CH₂)₂-OH | 6.6 |
| H₃C-CH₂-OH | 0 |
| H₃C-OH | 0 |
| glycerol | 0 |

As shown in Table 2, substrates for the enzyme of the present invention can be saturated or unsaturated aliphatic alcohols with more than three carbon atoms. When an ω-carboxylalcohol such as HO₂C-(CH₂)₁₁-OH and HO₂C-(CH₂)₁₅-OH is used, the aldehydes HO₂C-(CH₂)₁₁-CHO or HO₂C-(CH₂)₁₅-CHO, and dicarboxylic acids HO₂C-(CH₂)₁₁-CO₂H or HO₂C-(CH₂)₁₅-CO₂H are generated.
(4) Utilization of coenzyme:
No utilization of NAD and NADP is observed in the following assay method:
Assay method

| | |
|---|---|
| 0.2 M Tris-HCl buffer (pH 7.5) | 0.5 mℓ |
| 10 mM NAD or NADP | 0.2 mℓ |
| 10 mM 12-hydroxydodecanic acid | 0.2 mℓ |
| 1% Triton X-100 | 0.2 mℓ |
| water | 0.05 mℓ |

One unit of enzyme is added to the reaction mixture (2.0 mℓ) hereinabove and the absorbance at 340 nm at 37°C is continuously measured.
The result is shown in Table 3. No increase in the absorption is observed. The enzyme does not require NAD or NADP.

**TABLE 3**

| Time | A₃₄₀ₙₘ | |
|---|---|---|
| | NAD | NADP |
| 0 min | 0.062 | 0.046 |
| 2 min | 0.061 | 0.047 |
| 4 min | 0.060 | 0.045 |
| 6 min | 0.062 | 0.046 |
| 8 min | 0.061 | 0.046 |
| 10 min | 0.061 | 0.047 |

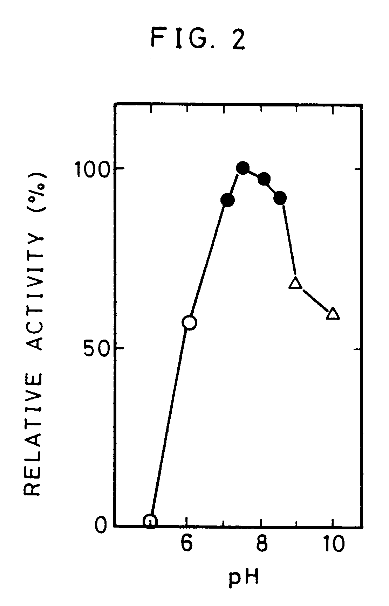

(5) Molecular weight:
Approximately 66000 (66000±6000), measured by the gel-filtration using a Superose 12 column.
(6) Optimum pH : pH 7.5 ± 0.5
The optimum pH is measured according to the assay method illustrated hereinbefore using the same reaction mixture, except that the pH is varied.
The results are shown in Fig. 2. In Fig. 2:
- ○: : MES-buffer
- ●: : Tris-HCl buffer,
- △: : glycine-NaOH buffer.

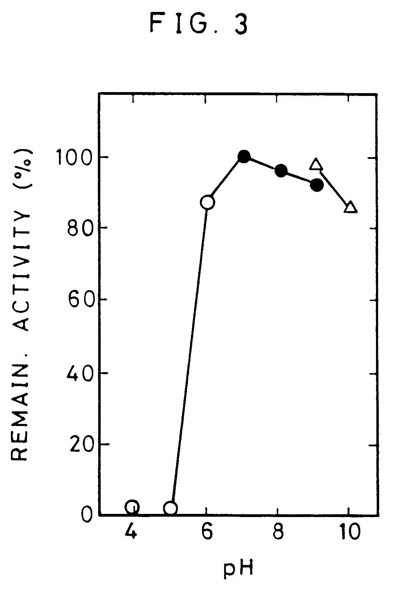

(7) Stable pH-range : pH 6 ∼ 9
The stable pH-range of the enzyme in various buffer solutions is measured by dissolving the enzyme in 10 mM buffer, heated at 37°C for 60 minutes and immediately cooled, and the remaining activities are measured at each pH.
The result is shown in Fig. 3, in which:
- ○: : MES-buffer
- ●: : Tris-HCl buffer,
- △: : glycine-NaOH buffer.

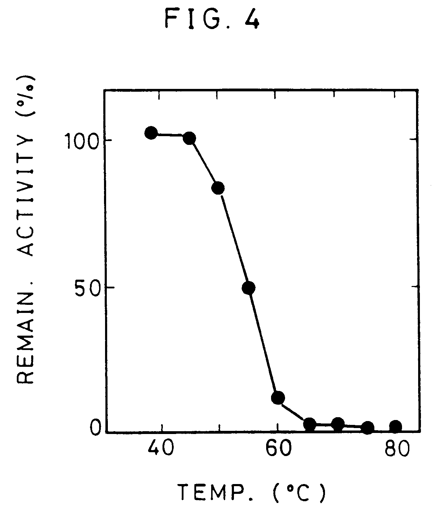

(8) Heat stability: Stable at 45°C (pH 7.5, 10 minutes).
The enzyme dissolved in 0.1 M Tris-HCl buffer (pH 7.5) is incubated at various temperatures for 10 minutes, and immediately cooled. The remaining activities are measured.
The results are shown in Fig. 4.
(9) Effects of reagents:
The effects of detergent and metal ions illustrated in Table 4 are shown in that Table. These reagents show no effect on the enzyme. Activities of the enzyme are measured by adding each detergent and metal ion.

**TABLE 4**

| Reagent | Concentration | Relative activity (%) |
|---|---|---|
| None | | 100 |
| NaCl | 100 mM | 95.1 |
| KCl | 100 mM | 97.6 |
| NH₄Cl | 100 mM | 89.0 |
| CaCl | 1 mM | 97.0 |
| MgCl₂ | 1 mM | 98.2 |
| MnCl₂ | 1 mM | 102.4 |
| Triton X-100 | 1.0% | 93.1 |
| Deoxycholate | 10 mM | 94.9 |
| Cholate | 10 mM | 94.9 |

(10) Isoelectric point: pH 4.5±0.2 (isoelectric focusing using carrier ampholite).

The present invention also provides a method for detecting an aliphatic alcohol, aliphatic aldehyde or ω-carboxylic acid derivative thereof of formula R'-CH₂OH or R'-CHO, wherein R'-CH₂OH is an aliphatic alcohol except methanol and ethanol or an ω-carboxylic acid derivative of said alcohol and R'-CHO is corresponding aliphatic aldehyde or an ω-carboxylic acid derivative thereof, in a sample, with an ω-carboxylalcohol oxidase as defined above and quantitatively determining the amount of a consumed or generated component.

The ω-carboxylalcohol oxidase used in the above method is not limited, so far as it has the biochemical properties shown hereinabove. The preferred enzyme is the ω-carboxylalcohol oxidase produced by Streptomyces sp. AC 8205.

R'-CH₂OH in a specimen is an aliphatic alcohol except methanol and ethanol or an ω-carboxylic acid derivative of said alcohol. For example it can be a long chain alcohol having three or more carbon atoms, such as HO₂C-(CH₂)₁₁-OH, H₂C-(CH₂)₁₅-OH, H₃C-(CH₂)₇-CH=CH-(CH₂)₈-OH, H₃C-(CH₂)₁₅-OH, H₃C-(CH₂)₁₃-OH, H₃C-(CH₂)₁₁-OH, H₃C-(CH₂)₉-OH, H₃C-(CH₂)₇-OH, H₃C-(CH₂)₅-OH, H₃C-(CH₂)₃-OH and H₃C-(CH₂)₂-OH. Among these, HO₂C-(CH₂)₁₁-OH, H₃C-(CH₂)₁₁-OH, H₃C-(CH₂)₉-OH, H₃C-(CH₂)₇-OH and H₃C-(CH₂)₅-OH are preferred.

R'-CHO in the specimen can be an aliphatic aldehyde except formaldehyde and acetaldehyde or an ω-carboxylic acid derivative of said aldehyde, for example the corresponding aldehyde compound of the aliphatic alcohol hereinabove or an ω-carboxylic acid derivative thereof.

The concentration of R'-CH₂OH or R'-CHO in a specimen is usually 0.01 to 0.05 mM. It may be diluted or subjected to a pretreatment. The reaction temperature is generally approximately 0 to 40°C. The amount of ω-carboxylalcohol oxidase used is approximately 1 to 10 units.

A component consumed in the reaction is a substrate component, oxygen or water. A generated component can be R'-CHO, R'-COOH or H₂O₂. In the case that R'-CH₂OH is an aliphatic alcohol except methanol and ethanol or an ω-carboxylic acid of an aliphatic alcohol except methanol and ethanol, a component, i.e. oxygen, R'-CHO, R'-COOH or H₂O₂, can be measured in the assay system.

In the case that R'-CHO is an aliphatic aldehyde except formaldehyde and acetaldehyde or the corresponding ω-carboxylic acid derivative of said aliphatic aldehyde, oxygen, R'-COOH or H₂O₂ can be measured in the assay system.

In an assay, any proper method can be used. For example, in the case that generated H₂O₂ is measured, the peroxidase method can be applied. A known chromogen, colouring reagent, luminescent reagent or fluorescence reagent are reacted with generated H₂O₂ in the presence of peroxidase and the absorbance of the reaction mixture is measured.

R'-CH₂OH or R'-CHO can be a component in the specimen as it is or can be a component generated from an aliphatic ester by the action of an esterase or lipase.

The present invention further provides a process for the production of a carboxylic acid, which comprises contacting, in an aqueous medium:
1) an ω-carboxylalcohol oxidase as defined above; and
2) R''-CH₂OH or R''-CHO, wherein R''-CH₂OH is an aliphatic alcohol except methanol and ethanol or an ω-carboxylic acid derivative of said alcohol, and R''-CHO is a corresponding aliphatic aldehyde or an ω-carboxylic acid derivative thereof, which alcohol or aldehyde is a substrate for said ω-carboxylalcohol oxidase and is a substrate which can produce a carboxylic acid;
   in the presence of oxygen.

The ω-carboxylalcohol oxidase used in the process for the production of a carboxylic acid is not limited, so far as it has the biochemical properties shown hereinbefore. A preferred enzyme is the ω-carboxylalcohol oxidase produced by Streptomyces sp. AC 8205.

The substrate R''-CH₂OH is an aliphatic alcohol, except methanol and ethanol, or an ω-carboxylic acid derivative of said alcohol. For example it can have a long chain of three or more carbon atoms, such as HO₂C-(CH₂)₁₁-OH, HO₂C-(CH₂)₁₅-OH, H₃C-(CH₂)₇-CH=CH-(CH₂)₈-OH, H₃C-(CH₂)₁₅-OH, H₃C-(CH₂)₁₃-OH, H₃C-(CH₂)₁₁-OH, H₃C-(CH₂)₉-OH, H₃C-(CH₂)₇-OH, H₃C-(CH₂)₅-OH, H₃C-(CH₂)₃ and H₃C-(CH₂)₂-OH. Among these, HO₂C-(CH₂)₁₁-OH, H₃C-(CH₂)₁₁-OH, H₃C-(CH₂)₉-OH, H₃C-(CH₂)₇-OH and H₃C-(CH₂)₅-OH are preferred. The substrate R''-CHO can be an aliphatic aldehyde except formaldehyde and acetaldehyde or an ω-carboxylic acid of said aldehyde, for example an aldehyde corresponding to the aliphatic alcohol hereinable or an ω-carboxylic acid derivative thereof.

The concentration thereof in the production is, for example, 10 to 500 »M.

The aqueous medium is not limited. It is a medium which contains water and has no detrimental effect on the enzymatic reaction. Examples are water or a buffer solution, and a mixed solvent of water and an organic solvent. Examples of organic solvents are methanol, ethanol, propanol or butanol, or acetone or mixtures thereof. Preferred examples are aqueous methanol, aqueous ethanol, aqueous acetone or chloroform.

Oxygen can be supplied by bubbling oxygen gas, or if the amount of the substrate is small, dissolved oxygen in an aqueous medium can be used for the reaction.

In the production of a carboxylic acid, the ω-carboxylalcohol oxidase of the present invention can be prepared as an immobilized enzyme with a carrier.

Immobilization can be carried out by a known method, for example a carrier binding method (binding with an insoluble carrier by a covalent bond, ionic bond or adsorption), cross linkage method or encapsulating method (lattice type polymer gel, microcapsules or hole fibers) or a combination thereof. A preferred method is a covalent bond method or adsorption method.

Examples of the carrier are a natural polymer, synthetic polymer and inorganic material, such as cellulose, agarose, dextran, chitin, collagen, tannin, fibrin, albumin, casein, carrageenan, amino acid polymer, polystyrene, polystyrene with a sulfonate group, polystyrene with a carboxyl group, polystyrene with an amino group, a copolymer of a substituted or unsubstituted styrene derivative monomer and methylstyrene, ethylstyrene , chlorstyrene, ethylene, propylene, acrylic acid, acrylic acid methyl ester, acrylic acid ethyl ester, methacrylic acid, methacrylic acid methyl ester, methacrylic acid ethyl ester, acrylonitrile, acrylamide, maleic acid, fumaric acid, butadiene, chloroprene, isoprene, vinylchloride, vinylidene chloride, vinylacetate, vinyl toluene or divinyl benzene, polyvinyl toluene, polyester, polyacrylate, polymethacrylate, polyacrylonitrile, aminated polyacrylonitrile, polyvinylpyrrolidone, polyacetate vinylacrylate and vinylchloride-acrylate copolymer.

Examples of the crosslinking reagent are glutaraldehyde, hexamethylene diisocyanate, toluene diisocyanate, xylene diisocyanate, dialdehyde starch, dimethyladipidate, dimethylsulbelimidate and dimethyl-3,3'-dithio bis propionimidate.

The carrier surface may be previously activated with hydrazine, thionylchloride, carbodiimide, phosgen, ethylene chloroformate or bromocyanide.

The form of the carrier may be selected according to the type of operation. It may be a pellet, granule, film, sheet, fiber or gel type carrier.

The reaction temperature is a temperature which does not denature the enzyme, for example approximately 10 to 40°C. The reaction time can be selected according to the conditions such as amount of enzyme used. It can be determined by checking the reaction process using TLC.

The enzyme activity used in the reaction is generally 1 to 10 units.

The present invention further provides Streptomyces sp AC 8205 FERM BP-2491 or a variant or mutant thereof which is able to produce an ω-carboxylalcohol oxidase as defined above.

An ω-dibasic acid, which is useful as a starting material for plastics and a lubricant, can be produced from the substrate such as an ω-carboxylic acid derivative of an aliphatic alcohol or an ω-carboxylic acid derivative of an aliphatic aldehyde. The enzymatic process of the present invention has an advantage as compared with chemical synthesis.

### EXAMPLES

The following Examples further illustrate the present invention.

### EXAMPLE 1

An aqueous medium (pH 7.0, 20ℓ) comprising peptone 1%, fish meat extract 1%, fructose 2%, NaCl 0.3%, KH₂PO₄ 0.3%, MgSO₄ 0.3% and antifoam reagent Disform BC-51Y 0.2% was sterilized at 120°C for 20 minutes. A seed culture (200 ml) of Streptomyces sp. AC 8205 FERM BP-2491 cultured previously in the same medium was inoculated into the above medium and submerged cultured at 28°C for 55 hours, with aseptic aeration of 20 ℓ/min. and 300 rpm agitation (0.01 U/mℓ).

The cultured broth (17 ℓ) was separated centrifugally at 5000 rpm for 10 minutes and the obtained cells were suspended in 10 mM phosphate buffer (pH 7.0, 8 ℓ) containing 0.1% Triton X-100 and lysozyme at 1 mg/mℓ, then autolysated at 37°C for 2 hours. The mixture was centrifuged at 5000 rpm for 10 minutes to obtain crude enzyme extract solution (7.8 ℓ). Acetone (2-fold volumes) was added thereto and the precipitate was dissolved in 10 mM phosphate buffer (pH 7.0) containing 20% ammonium sulfate.

Insoluble material was removed by centrifugation at 12000 rpm for 10 minutes. The supernatant solution (490 mℓ) was charged on an octyl-Sepharose CL-6B column (2.6 x 10 cm) to adsorb the enzyme. Then the column was subjected to elution with 15% ammonium sulfate solution (300 mℓ) and 10% ammonium sulfate solution (300 mℓ), and the active fractions measured according to the assay method, i.e. fractions (300 mℓ) eluted by 10% ammonium sulfate were collected and dialysed in a cellulose acetate tube against 10 mM Tris-HCl buffer (pH 7.5, 5 ℓ) at 4°C for 15 hours. The dialysate was lyophilized to obtain purified ω-carboxylalcohol oxidase (155 mg, 0.3 U/mg).

### EXAMPLE 2

Assaying ω-carboxylic acid of aliphatic alcohol

| | |
|---|---|
| 0.2 M Tris-HCl buffer (pH 8.0) | 0.5 mℓ |
| 0.3% 4-aminoantipyrine | 0.3 mℓ |
| 0.2% phenol | 0.3 mℓ |
| peroxidase (45 U/mℓ) | 0.2 mℓ |
| ω-carboxylalcohol oxidase (5 U/mℓ) | 0.1 mℓ |
| water | 0.6 mℓ |

12-hydroxydodecanic acid (20 »ℓ) was added to the above reaction mixture, incubated at 37°C for 10 mins and then absorbance at 500 nm was measured.

The results are shown in Fig. 5. A good standard curve was obtained.

### EXAMPLE 3

Production of dibasic acid:
The enzyme produced in Example 1 was dissolved in 0.2 M PIPES-NaOH buffer (pH 7.3, 0.5 mℓ). The said enzyme solution (2U) and catalase (bovine liver, 5U) were added to 12-hydroxydodecanic acid (100 mg) dissolved in acetone (10 mℓ) and reacted at 28°C for 18 hours.

The dried reaction mixture was mixed with chloroform-methanol (2 : 1) (5 mℓ) and water (2 mℓ). The chloroform layer was dried to obtain the dibasic acid, 1,12-dodecane dicarboxylic acid (75 mg).
- TLC :: Rf = approx 0.8 [silica gel 60 plate, developer : chloroform-methanol-acetone (80 : 20 : 5), detection : 0.03% methyl red-96% ethanol solution]

### EXAMPLE 4

Example 3 was repeated except that the acetone solution of 12-hydroxydodecanic acid was replaced by 16-hydroxy hexadecanic acid (100 mg) dissolved in chloroform (10 mℓ) and reaction time was 15 hours to obtain 1,16-hexadecane dicarboxylic acid (72 mg).

### EXAMPLE 5

Production of carboxylic acid using immobilized enzyme:
(1) Immobilization of the enzyme:
Aminated polyacrylnitrile fibers (2.0 g, wet weight) were treated with 25% glutaraldehyde at 0°C for 1 hour, filtered with a glass filter and washed with purified water (200 mℓ).
The enzyme (25 mℓ, 30 units) dissolved in 1M phosphate buffer (pH 7.0) was contacted with the fibers, stirred at 30°C for 30 mins, filtered with glass filters, and washed with 10mM Tris-HCl buffer (50 mℓ) to obtain the immobilized enzyme (2.1 g).

| | |
|---|---|
| Immobilization ratio: | 73% |
| Activity expressed: | 67% |

(2) Production of carboxylic acid with immobilized enzyme system:
Immobilized enzyme (200 mg) obtained in (1) hereinabove was used in the same conditions as in Example 4. Dibasic acid, 1,16-hexadecane dicarboxylic acid (80 mg), was produced.

The present invention provides a novel enzyme, ω-carboxyalcohol oxidase. The enzyme is useful for the analysis and assay of various alcohols and as a diagnostic enzyme reagent. Furthermore, a carboxylic acid can be produced using the enzyme from an alcohol or aldehyde. Specifically, a dibasic acid can be produced from ω-carboxylic acids of aliphatic alcohols or aldehydes. Among dibasic acids, a C₆₋₁₅ dibasic acid is used as a raw material, for example, as a monomer for a plastics polymer, synthetic fibers such as nylon, antifreeze plasticizer or lubricant. Examples are brassilic acid and pentadecanedioic acid, in which when the former is esterified to prepare a polyester and is aminated to produce a polyamide. These can be used for synthetic plastics, synthetic lubricants, plasticizers or perfumes. The latter is used as a raw material of the neutral perfume cyclopentadecanone. These materials are difficult to produce by chemical synthesis with low cost and hence the present invention provides useful production processes.

Furthermore, the assay method of an aliphatic alcohol and aliphatic aldehyde or an ω-carboxylic acid derivative thereof of the present invention can be made quantitatively and precisely. Since an ω-carboxylic acid derivative of an aliphatic alcohol is an intermediate for an ω-hydroxylated fatty acid, the assay method of the present invention is useful for an intermediate metabolite of a fatty acid and for screening for the enzyme in the metabolism of a fatty acid.

## Claims

1. An ω-carboxylalcohol oxidase having at least the following biochemical properties:
1) Enzyme action: at least catalysing the following reactions a) and b):
a) R-CH₂OH + O₂ → R-CHO + H₂O₂
b) R-CHO + O₂ + H₂O → R-COOH + H₂O₂
wherein R-CH₂OH is an aliphatic alcohol except methanol and ethanol or an ω-carboxylic acid derivative of said alcohol, R-CHO is a corresponding aliphatic aldehyde or an ω-carboxylic acid derivative thereof, and R-COOH is a corresponding compound thereto;
2) substrate specificity: at least having substrate specificity for HO₂C-(CH₂)₁₁-OH, H₃C-(CH₂)₁₁-OH, H₃C-(CH₂)₉-OH, H₃C-(CH₂)₇-OH and H₃C-(CH₂)₅-OH, and having no substrate specificity for methanol, ethanol and glycerol; and
3) utilization of coenzyme: no utilization of NAD and NADP.

2. An ω-carboxyalcohol oxidase according to claim 1 having the biochemical properties:
- molecular weight : 66000 ± 6000 (gel filtration method using a Superose 12 column),
- optimum pH : 7.5 ± 0.5
- stable pH range : pH 6 ∼ 9
- heat stability : stable at 45°C (at pH 7.5, 10 minutes).

3. A process for production of an ω-carboxyalcohol oxidase as defined in claim 1 or 2 which comprises culturing a said ω-carboxylalcohol oxidase producing microorganism belonging to the genus Streptomyces in a medium, and isolating said ω-carboxylalcohol oxidase therefrom.

4. A process according to claim 3 wherein the microorganism is Streptomyces sp. AC 8205 FERM BP-2491 or a variant or mutant thereof which has an ability to produce an isolatable amount of ω-carboxylalcohol oxidase.

5. A method for detecting an aliphatic alcohol, aliphatic aldehyde or ω-carboxylic acid derivative thereof of formula R'-CH₂OH or R'-CHO, wherein R'-CH₂OH is an aliphatic alcohol except methanol and ethanol or an ω-carboxylic acid derivative of said alcohol and R'-CHO is a corresponding aliphatic aldehyde or an ω-carboxylic acid derivative thereof, in a sample, comprising reacting, in an aqueous medium, the sample with an ω-carboxylalcohol oxidase as defined in claim 1 or 2, and quantitatively determining the amount of a consumed or generated component.

6. A method according to claim 5 wherein R'-CH₂OH is an aliphatic alcohol except methanol and ethanol, a consumed component in the reaction is oxygen, a generated component is R'-CHO, R'-COOH or H₂O₂, and a component which is determined is oxygen, R'-CHO, R'-COOH or H₂O₂.

7. A method according to claim 5 wherein R'-CH₂OH is an ω-carboxylic acid derivative of an aliphatic alcohol except methanol and ethanol, a consumed component in the reaction is oxygen, a generated component is R'-CHO, R'-COOH or H₂O₂, and a component which is determined is oxygen, R'-CHO, R'-COOH or H₂O₂.

8. A method according to claim 5 wherein R'-CHO is an aliphatic aldehyde except formaldehyde and acetaldehyde, a consumed component in the reaction is oxygen, a generated component is R'-COOH or H₂O₂, and a component which is determined is oxygen, R'-COOH or H₂O₂.

9. A method according to claim 5 wherein R'-CHO is an ω-carboxylic acid derivative of an aliphatic aldehyde except formaldehyde and acetaldehyde, a consumed component in the reaction is oxygen, a generated component is R'-COOH or H₂O₂, and a component which is determined is oxygen, R'-COOH or H₂O₂.

10. A method according to any one claims 5 to 9 wherein R'-CH₂OH or R'-CHO originally exists in the sample or is a generated component.

11. A process for the production of a carboxylic acid, which comprises contacting, in an aqueous medium:
1) an ω-carboxylalcohol oxidase as defined in claim 1 or 2; and
2) R''-CH₂OH or R''-CHO, wherein R''-CH₂OH is an aliphatic alcohol except methanol and ethanol or an ω-carboxylic acid derivative of said alcohol, and R''-CHO is a corresponding aliphatic aldehyde or an ω-carboxylic acid derivative thereof, which alcohol or aldehyde is a substrate for said ω-carboxylalcohol oxidase and is a substrate which can produce a carboxylic acid;
in the presence of oxygen.

12. A process according to claim 11 wherein R''-CH₂OH or R''-CHO is an ω-carboxylic acid derivative of an aliphatic alcohol or an ω-carboxylic acid derivative of an aliphatic aldehyde, and the produced carboxylic acid is a dibasic acid.

13. Streptomyces sp AC 8205 FERM BP-2491 or a variant or mutant thereof which is able to produce an ω-carboxylalcohol oxidase as defined in claim 1 or 2.

## Patentansprüche

1. Eine omega-Carboxylalkoholoxidase mit mindestens den folgenden biochemischen Eigenschaften:
(1) Enzymwirkung: Katalysiert zumindest die folgenden Reaktionen (a) und (b):
(a) R-CH₂OH + O₂ → R-CHO + H₂O₂
(b) R-CHO + O₂ + H₂O → R-COOH + H₂O₂
wobei R-CH₂OH einen aliphatischen Alkohol mit Ausnahme von Methanol und Ethanol oder eine omega-Carbonsäurederivat des Alkohols darstellt, R-CHO ein korrespondierender aliphatischer Aldehyd oder sein omega-Carbonsäurederivat ist und R-COOH eine dazu korrespondierende Verbindung ist;
(2) Substratspezifität: Mindestens Substratspezifität für HO₂C-(CH₂)₁₁-OH, H₃C-(CH₂)₁₁-OH, H₃C-(CH₂)₉-OH, H₃C-(CH₂)₇-OH und H₃C-(CH₂)₅-OH und keine Substratspezifität für Methanol, Ethanol und Glyzerin; und
(3) Erfordernis eines Koenzyms: Kein Erfordernis für NAD und NADP.

2. Eine omega-Carboxylalkoholoxidase nach Anspruch 1 mit den folgenden biochemischen Eigenschaften:
- Molekulargewicht: 66000 ± 6000 (Gelfiltrationsmethode mit einer Superose-12-Säule),
- optimaler pH-Wert: 7,5 ± 0,5
- stabiler pH-Bereich: etwa pH 6 bis 9
- Wärmestabilität: Stabil bei 45 °C (bei pH 7,5 10 min).

3. Verfahren zur Herstellung einer omega-Carboxylalkoholoxidase gemäß Anspruch 1 oder 2, bei dem man einen die omega-Carboxylalkoholoxidase produzierenden Mikroorganismus der Gattung *Streptomyces* in einem Medium kultiviert und die omega-Carboxylalkoholoxidase isoliert.

4. Verfahren nach Anspruch 3, bei dem man als Mikroorganismus *Streptomyces* sp. AC 8205 FERM BP-2491 oder eine seiner Varianten oder Mutanten verwendet, die eine isolierbare Menge an omega-Carboxylalkoholoxidase produzierten kann.

5. Verfahren zur Ermittlung eines aliphatischen Alkohols, eines aliphatischen Aldehyds oder eines seiner omega-Carbonsäurederivate der Formel R'-CH₂OH oder R'-CHO, wobei R'-CH₂OH ein aliphatischer Alkohol mit Ausnahme von Methanol und Ethanol oder ein omega-Carbonsäurederivat des Alkohols ist und R'-CHO ein korrespondierender aliphatischer Aldehyd oder eines seiner omega-Carbonsäurederivate ist, in einer Probe, wobei man in einem wäßrigen Medium die Probe mit einer omega-Carboxylalkoholoxidase gemäß Anspruch 1 oder 2 umsetzt und quantitativ die Menge an verbrauchter oder erzeugter Komponente bestimmt.

6. Verfahren nach Anspruch 5, bei dem R'-CH₂OH einen aliphatischen Alkohol mit Ausnahme von Methanol und Ethanol darstellt, eine Komponente, die bei der Umsetzung verbraucht wird, Sauerstoff ist, eine erzeugte Komponente R'-CHO, R'-COOH oder H₂O₂ ist und eine zu bestimmende Komponente Sauerstoff, R'-CHO, R'-COOH oder H₂O₂ ist.

7. Verfahren nach Anspruch 5, bei dem R'-CH₂OH ein omega-Carbonsäurederivat eines aliphatischen Alkohols mit Ausnahme von Methanol und Ethanol ist, eine Komponente, die bei der Reaktion verbraucht wird, Sauerstoff ist, eine erzeugte Komponente R'-CHO, R'-COOH oder H₂O₂ ist und eine zu bestimmende Komponente Sauerstoff, R'-CHO, R'-COOH oder H₂O₂ ist.

8. Verfahren nach Anspruch 5, bei dem R'-CHO ein aliphatischer Aldehyd mit Ausnahme von Formaldehyd und Acetaldehyd ist, eine Komponente, die bei der Reaktion verbraucht wird, Sauerstoff ist, eine erzeugte Komponente R'-COOH oder H₂O₂ ist und eine zu bestimmende Komponente Sauerstoff, R'-COOH oder H₂O₂ ist.

9. Verfahren nach Anspruch 5, bei dem R'-CHO ein omega-Carbonsäurederivat eines aliphatischen Aldehyds mit Ausnahme von Formaldehyd und Acetaldehyd ist, eine Komponente, die bei der Umsetzung verbraucht wird, Sauerstoff ist, eine erzeugte Komponente R'-COOH oder H₂O₂ ist und eine zu bestimmende Komponente Sauerstoff, R'-COOH oder H₂O₂ ist.

10. Verfahren nach einem der Ansprüche 5 bis 9, bei dem R'-CH₂OH oder R'-CHO ursprünglich in der Probe vorliegt oder eine erzeugte Komponente ist.

11. Verfahren zur Herstellung einer Carbonsäure, bei dem man in einem wäßrigen Medium
(1) eine omega-Carboxylalkoholoxidase gemäß Anspruch 1 oder 2 und
(2) R''-CH₂OH oder R''-CHO, wobei R''-CH₂OH ein aliphatischer Alkohol mit Ausnahme von Methanol und Ethanol oder ein omega-Carbonsäurederivat des Alkohols ist und R''-CHO ein korrespondierender aliphatischer Aldehyd oder eines seiner omega-Carbonsäurederivate ist, wobei der Alkohol oder der Aldehyd ein Substrat für die omega-Carboxylalkoholoxidase und ein Substrat sind, das eine Carbonsäure bildet,
in Gegenwart von Sauerstoff in Berührung bringt.

12. Verfahren nach Anspruch 11, bei dem R''-CH₂OH oder R''-CHO ein omega-Carbonsäurederivat eines aliphatischen Alkohols oder ein omega-Carbonsäurederivat eines aliphatischen Aldehyds sind und die gebildete Carbonsäure eine zweibasische Säure ist.

13. *Streptomyces* sp. AC 8205 FERM BP-2491 oder eine seiner Varianten oder Mutanten, die eine omega-Carboxylalkoholoxidase gemäß Anspruch 1 oder 2 bilden kann.

## Revendications

1. ω-Carboxylalcool oxydase, ayant au moins les propriétés biochimiques qui suivent :
1) Action d'enzyme : catalysant au moins les réactions a) et b) qui suivent :
a) R-CH₂OH + O₂ → R-CHO + H₂O₂
b) R-CHO +O₂ + H₂O → R-COOH + H₂O₂
où R-CH₂OH est un alcool aliphatique à l'exception du méthanol et de l'éthanol ou un dérivé d'acide ω-carboxylique dudit alcool, R-CHO est un aldéhyde aliphatique correspondant ou son dérivé d'acide ω-carboxylique, et R-COOH est un composé correspondant;
2) Spécificité du substrat : ayant au moins une spécificité de substrat pour HO₂C-(CH₂)₁₁-OH, H₃C-(CH₂)₁₁-OH, H₃C-(CH₂)₉-OH, H₃C-(CH₂)₇-OH et H₃C-(CH₂)₅-OH, et n'ayant pas de spécificité du substrat pour le méthanol, l'éthanol ni le glycérol; et
3) Utilisation de la coenzyme : pas d'utilisation de NAD ni de NADP.

2. ω-Carboxyalcool oxydase selon la revendication 1, ayant les propriétés biochimiques :
Poids moléculaire : 66000 ± 6000 (méthode de filtration sur gel en utilisant une colonne de Superose 12),
pH optimum : 7,5 ± 0,5
plage de pH stable: pH 6 - 9
Stabilité à la chaleur : stable à 45°C (à pH 7,5, 10 minutes).

3. Procédé de production d'une ω-carboxyalcool oxydase, telle que définie à la revendication 1 ou 2, qui comprend la mise en culture d'un microorganisme produisant ladite ω-carboxylalcool oxydase, appartenant au genre Streptomyces dans un milieu, et l'isolement de ladite ω-carboxylalcool oxydase.

4. Procédé selon la revendication 3, où le microorganisme est Streptomyces sp. AC 8205 FERM BP-2491 ou son variant ou mutant, qui a l'aptitude de produire une quantité d'ω-carboxylalcool oxydase pouvant être isolée.

5. Méthode de détection d'un alcool aliphatique, d'un aldéhyde aliphatique ou de son dérivé d'acide ω-carboxylique de formule R'-CH₂OH ou R'-CHO, où R'-CH₂OH est un alcool aliphatique, à l'exception du méthanol et de l'éthanol ou un dérivé d'acide ω-carboxylique dudit alcool et R'-CHO est un aldéhyde aliphatique correspondant ou son dérivé d'acide ω-carboxylique, dans un échantillon, comprenant la réaction, dans un milieu aqueux, de l'échantillon avec une ω-carboxylalcool oxydase telle que définie à la revendication 1 ou 2, et la détermination quantitative de la quantité d'un composant consommé ou produit.

6. Méthode selon la revendication 5, où R'-CH₂OH est un alcool aliphatique à l'exception du méthanol et de l'éthanol, un composant consommé dans la réaction est l'oxygène, un composant produit est R'-CHO, R'-COOH ou H₂O₂, et un composant qui est déterminé est l'oxygène, R'-CHO, R'-COOH ou H₂O₂.

7. Méthode selon la revendication 5, où R'-CH₂OH est un dérivé d'acide ω-carboxylique d'un alcool aliphatique, à l'exception du méthanol et de l'éthanol, un composant consommé dans la réaction est l'oxygène, un composant produit est R'-CHO, R'-COOH ou H₂O₂, et un composant qui est déterminé est l'oxygène, R'-CHO, R'-COOH ou H₂O₂.

8. Méthode selon la revendication 5, où R'-CHO est un aldéhyde aliphatique, à l'exception du formaldéhyde et de l'acétaldéhyde, un composant consommé dans la réaction est l'oxygène, un composant produit est R'-COOH ou H₂O₂ et un composant qui est déterminé est l'oxygène, R'-COOH ou H₂O₂.

9. Méthode selon la revendication 5, où R'CHO est un dérivé d'acide ω-carboxylique ou un aldéhyde aliphatique, à l'exception du formaldéhyde et de l'acétaldéhyde, un composant consommé dans la réaction est l'oxygène, un composant produit est R'-COOH ou H₂O₂ et un composant qui est déterminé est l'oxygène, R'-COOH ou H₂O₂.

10. Méthode selon l'une quelconque des revendications 5 à 9, où R'-CH₂OH ou R'-CHO existe à l'origine dans l'échantillon ou est un composant produit.

11. Procédé de production d'un acide carboxylique qui comprend la mise en contact, dans un milieu aqueux :
1) d'une ω-carboxylalcool oxydase, telle que définie à la revendication 1 ou 2; et
2) de R''-CH₂OH ou R''-CHO, où R''-CH₂OH est un alcool aliphatique à l'exception du méthanol et de l'éthanol, ou bien un dérivé d'acide ω-carboxylique dudit alcool, et R''-CHO est un aldéhyde aliphatique correspondant ou son dérivé d'acide ω-carboxylique, lequel alcool ou aldéhyde est un substrat pour ladite ω-carboxylalcool oxydase et est un substrat qui peut produire un acide carboxylique;
en présence d'oxygène.

12. Procédé selon la revendication 11, où R''-CH₂OH ou R''-CHO est un dérivé d'acide ω-carboxylique d'un alcool aliphatique ou un dérivé d'acide ω-carboxylique d'un aldéhyde aliphatique , et l'acide carboxylique produit est un acide dibasique.

13. Streptomyces sp AC 8205 FERM BP-2491 ou son variant ou mutant, qui peut produire une ω-carboxylalcool oxydase, telle que définie à la revendication 1 ou 2.
